Europäisches Patentamt

(19) European Patent Office

Office européen des brevets

(11) Publication number: 0 111 928

A1

## (12) EUROPEAN PATENT APPLICATION

(21) Application number: 83112839.2

(22) Date of filing: 20.12.83

(51) Int. Cl.³: **C 07 C 85/06**
C 07 D 295/02, C 07 D 295/12
C 07 D 307/08, C 07 D 319/12
C 07 D 487/08, B 01 J 27/18
//(C07D487/08, 241/00, 241/00)

(30) Priority: 20.12.82 US 451295
20.12.82 US 451305

(43) Date of publication of application:
27.06.84 Bulletin 84/26

(84) Designated Contracting States:
AT BE CH DE FR GB IT LI LU NL SE

(71) Applicant: AIR PRODUCTS AND CHEMICALS, INC.
Route no. 222
Trexlertown Pennsylvania 18087(US)

(72) Inventor: Wells, James Edward
2115 Bryn Mawr Place
Ardmore, PA 19003(US)

(72) Inventor: Eskinazi, Victoria
3360 Chichester Avenue Apartment P-11
Boothwyn, PA 19061(US)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) Catalysis of condensation reactions.

(57) Certain hydrogen phosphate and pyrophosphate compositions are employed as catalysts for organic condensation reactions. Particularly high conversions and selectivities are obtained by the use of synergistic mixtures of in cyclization reactions such as in the conversion of hydroxyethylpiperazine to triethylenediamine and morpholine to dimethylaminoethylmorpholine.

EP 0 111 928 A1

# CATALYSIS OF CONDENSATION REACTIONS

## TECHNICAL FIELD OF THE INVENTION

The present invention relates to organic condensation reactions effected in the presence of novel pyrophosphate and hydrogen phosphate catalysts and is more particularly concerned with the production of amine compounds in enhanced yields.

## BACKGROUND OF THE PRIOR ART

Organic synthesis by condensation reactions resulting in the loss of a molecule of water or of ammonia are well known in the art. Certain of such reactions are generally effected in the presence of acidic catalysts. An important area in which such acid catalysis has been employed is in cyclization reactions as in the synthesis of triethylenediamine and its C-substituted homologues. The catalysts more generally used or proposed for use in such cyclization reactions are solid products of the Lewis acid type.

Triethylenediamine, also called diazabicyclo -[2.2.2]- octane, has been widely employed commercially as a catalyst in organic isocyanate reactions with compounds containing labile hydrogen, as in the production of urethane polymers. Triethylenediamine (sometimes hereinafter referred to as TEDA) was initially prepared in significant quantities by methods such as that described in U.S. Patent No. 2,937,176, by passing aliphatic amines in vapor phase over acidic cracking catalyst, such as silica-alumina dried gel or acid-activated clays. Numerous other feed stocks as well as other catalysts are disclosed in subsequent patents for preparation of TEDA as well as C-alkyl derivatives thereof.

Typical among these are U.S. Patents 2,985,658 and 3,166,558 employing preferably silica-alumina type catalyst, but listing also other useful solid acid catalysts that can be employed such as alumina in which phosphate or fluoride ion is incorporated (U.S. 2,985,658). It is disclosed in U.S. 3,116,558 that while these reactions can take place under superatmospheric pressures, they offer no advantage over atmospheric or subatmospheric pressures.

Among other catalysts proposed in the patent art for preparation of triethylene diamine and/or C-alkyl homologues thereof, are certain phosphate compounds, particularly aluminum phosphate.

The use of aluminum phosphate as a catalyst in the preparation of heterocyclic compounds from aliphatic amines was early disclosed in U.S. Patent 2,467,205, particularly for the preparation of piperazine from ethylenediamine or from polyethylene polyamine. The use of aluminum phosphate as catalyst in the preparation of triethylenediamine accompanied by piperazine among other by-products is further described in U.S. Patent 3,172,891; while U.S. Patent 3,342,820 describes the use of complex phosphates of alkali metal and trivalent metals in the preparation of C-alkyl TEDA.

U.S.S.R. Inventor's Certificate No. 525,681 discloses that the known processes for the preparation of TEDA by the catalytic conversion of various amines use aluminosilicate cracking catalysts, alumina with different additives, tungsten oxide or phosphates of metals at temperatures of 220°C to 550°C, under pressures of 0,1 to 150 atm. (absolute), in the presence of various diluents.

U.S. Patent 3,297,701 discloses as catalysts for preparation of TEDA and C-alkyl TEDA, in addition to the preferred aluminum phosphate stated to be superior, other phosphate compounds including calcium and iron phosphates among other listed metal phosphates. In the conversion of N-aminoethylpiperazine to triethylenediamine over aluminum phosphate catalyst, at most up to 39 mol% triethylenediamine is said to be obtained. Other of the named metal phosphate catalysts in the examples of the patent obtain yields of less than 10 mol% TEDA.

Acid metal phosphate catalysts, particularly phosphates of boron, aluminum and trivalent iron, have also been proposed for use in intramolecular cyclic dehydration reactions and other condensation reactions involving amino compounds. Examples of such reactions are found in U.S. Patent 4,117,227, which discloses conversion of an N-substituted diethanolamine to the corresponding N-substituted morpholine.

These condensation reactions are carried out in liquid phase at a temperature of from about 190°C to about 260°C and at any pressure to maintain reactants in a liquid state, i.e. generally from about 10 to about 1000 psig. U.S. Patent 4,036,881 describes preparation of non-cyclic polyalkylene polyamines by condensation of an alkylene diamine with an ethanolamine. N-hydroxethylmorpholine is condensed with morpholine in the presence of aluminum phosphate catalyst at temperatures from about 240° to 300°C and pressures of about 200 to 500 psig to form dimorpholino ethane according to U.S. Patent 4,103,087. Similarly, dimorpholinodiethyl ether is obtained by condensation of hydroxyethyl morpholine with aminoethyl morpholine over iron, aluminum or boron phosphate in U.S. Patent 4,095,022. Reaction of piperazine with ethanolamine over such acidic metal phosphate at a temperature of from about 250° to about 350°C and a pressure ranging from about 200 psig to about 500 psig produces N-aminoethyl piperazine

0111928

according to U.S. Patent 4,049,657. U.K. Patent 1,492,359 discloses the preparation of morpholine compounds by reacting an aminoalkoxyalkanol compound over phosphoric acid and similar types of phosphorus-containing substances.

Pyrophosphates of lithium, sodium, strontium and barium have been used as dehydration catalysts; see U.S. Patent 3,957,900. Phosphates and pyrophosphates of strontium and nickel have been used for the dehydrogenation of, for example, n-butene to butadiene under the conditions described in U.S. Patent 3,541,172.

It is also known to manufacture amines from ammonia and an alcohol over alumina, silica-alumina, silica, titania, tungstic oxides, clays or various metal phosphates of the typ referred to above at ammonia to alcohol mole ratios of from 2:1 to 6:1 depending on the ratio of amines desired, temperatures in the range of 300°C to 500°C pressures of 790 to 3550 kilopascals (100 - 500 psig), and a gas hourly space velocity of 500 to 1500 mol/mol; see Kirk-Othmer Encyclopedia of Chemical Technology, third Edition, Vol. 2 (1978), page 276.

## SUMMARY OF THE INVENTION

It has now been found that high yields of organic compounds are selectively obtained when the condensation reaction thereof is carried out in the presence of

5

catalytic amounts of a catalyst selected from the group consisting of the pyrophosphate, monohydrogen phosphate and dihydrogen phosphate of strontium, copper, magnesium, calcium, barium, zinc, aluminum, lanthanum, cobalt, nickel, cerium, neodymium, and mixtures thereof.

## DETAILED DESCRIPTION OF THE INVENTION

The monohydrogen and dihydrogen phosphate catalysts of the present invention are prepared by reaction of a mono- or diphosphate of an alkali metal or ammonium with a soluble salt of strontium, copper, magnesium, calcium, barium, zinc, aluminum, lanthanum, cobalt, nickel, cerium or neodymium at ambient temperatures. The highest purity and best yields of the present invention are obtained when using the soluble metal salts of a strong acid such as the metal nitrates, in substantially stoichiometric proportion to the phosphate. In aqueous media under these conditions, the reaction mixture is at a pH of about 3.5 to 6.5. In general, to obtain a precipitate of desired high content of the metal monohydrogen or dihydrogen phosphate, the ratio of phosphate to metal salt in the reaction mixture should be such as to have a pH of 5 ± 3, or the mixture should be adjusted to that pH range.

The pyrophosphate form of the catalysts of the present invention are prepared by heat treating the metal monohydrogen or dihydrogen phosphate product at temperatures above about 300°C up to 750°C in the presence of a mixture of steam and air, preferably at least about 20% by volume of steam.

For use as a catalyst, the metal pyro-, monohydrogen or dihydrogen phosphate product may be employed in the form of irregular particles of the desired size range prepared by breaking up the washed and dried filter cake or in the form of regular shaped pellets obtained by known methods of casting or extruding or the product may be deposited or otherwise impregnated into the pores of a microporous substrate such as alumina, silica, silica-alumina, and the like. In using the catalyst of the present invention to catalyze organic condensation reactions, substantially the same conditions may be employed as when using the known catalysts for the particular synthesis. For optimum results, however, some adjustment in temperature, diluent and/or space rate may be found beneficial.

Some specific examples of the type of organic compounds selectively obtained by the method of this invention include TEDA, the aliphatic alkylamines such as methylamine, methylethylamine, dimethylethylamine, morpholine, and dimethylaminoethylmorpholine. In the production of these compounds, the temperature is in the range of about 285° to 420°C, the pressure is in the range of above 1,5 and up to 150 atmospheres, and the liquid hourly space velocity (LHSV) of the organic feed stock per volume of catalyst is in the range of about 0.05 to 1.5. Preferably depending on the particular reaction, the temperature is in the range of about 300° to 400°C, the pressure is in the range of about 3 to 10 atmospheres and the LHSV is in the range of about 0.1 to 0.3 to obtain the highest yields and most economical process. The operable ratio of the organic feeds to water diluent is about 10 to 90% on a weight basis and preferably, 20 to 80% by weight. The optimum yield of these compounds is likely to be obtained using the highest temperature in the preferred range at the lowest LHSV.

In the preparation of TEDA, the preferred catalyst is selected from the group consisting of monohydrogen phosphate of calcium, magnesium, zinc, mixtures of strontium and barium in the ratio of Sr to Ba of about 1 to 5 to 5 to 1 and mixtures of lanthanum and strontium in the ratio of La to Sr of about 15 to 1 to 15. The organic feed stock used in this reaction to produce

TEDA is a substituted piperazine compound selected from the group consisting of hydroxyethylpiperazine and aminoethylpiperazine. The catalysts of this invention are relatively uneffected by the purity of the feed stock. For example, high conversion and good yields can be obtained from crude hydroxyethylpiperazine which contains minor quantities of piperazine and bis hydroxyethylpiperazine.

In the preparation of dimethylaminoethylmorpholine (DMAEM), the preferred catalyst is a mixture of strontium and nickel monohydrogen phosphate in the ratio of Sr to Ni of about 1 to 5 to 5 to 1. The feed stock is morpholine and dimethylethanolamine in the molar ratio in the range of about 1 to 3 and 3 to 1. Preferably, the reaction takes place in the presence of hydrogen in the molar ratio of hydrogen to organic feed of about 1 to 1 to 20 to 1 and an inert gas such as nitrogen, argon or helium in the molar ratio of inert gas to organic feed of about 1 to 1 to 20 to 1.

In the preparation of morpholine compounds, e.g. morpholine and alkyl morpholine, wherein the alkyl group has from 1 to 6 carbon atoms, diglycolamine compounds, e.g. diglycolamine and alkyl diglycolamines, wherein the alkyl group has from 1 to 6 carbon atoms are preferably carried out in the presence of strontium monohydrogen phosphate at about 300 to 370°C with the other conditions remaining the same as that discussed above. This reaction also preferably takes place in the presence of the inert gas in ratios of 2 to 1 to 10 to 1 inert gas to liquid organic feed stock.

The methods and catalysts of this invention are also capable of reacting an alcohol and a nitrogen-containing compound selected from the group consisting of ammonia, aliphatic primary and secondary amines, and aromatic primary and secondary amines to selectively convert this compound to the corresponding symmetrical or unsymmetrical higher molecular weight amine with little, if any, conversion to the corresponding by-products of thermodynamic amine equilibration. The amines and alcohol in the feed stock each contain 1 to 20 carbons per molecule. Preferably, the catalyst is lanthanum or copper monohydrogen phosphate and the molar ratio of alcohol to nitrogen-containing compound ranges from about 1 to 6 to 6 to 1.

## CATALYST PREPARATION

### Example 1

200 grams of strontium nitrate [$Sr(NO_3)_2$] was dissolved in distilled water and brought to a total volume of 800 cc with distilled water. To this solution there was added 10 cc of 85% phosphoric acid followed by 34.5 cc of 50% sodium hydroxide added rapidly with vigorous stirring. The resultant fine white precipitate was stirred for 10 minutes, vacuum-filtered and water-washed. The obtained filter cake was air dried in a static oven at approximately 110°C and extruded into 1/8 inch pellets for evaluation.

The obtained product had a surface area of 10-15 $m^2/g$. By X-ray diffraction the principal component was identified as $\beta$-$SrHPO_4$ with minor quantities of $Sr_5(OH)(PO_4)_3$ and unreacted $Sr(NO_3)_2$. Infrared spectroscopy showed a spectrum consistent with $SrHPO_4$. (Ref: Richard A. Nygurst and Ronald O. Kagel, "Infrared spectra of Inorganic Compounds", page 163, 1971).

### Example 2

195 grams of barium nitrate --$Ba(NO_3)_2$-- and 53 grams of strontium nitrate --$Sr(NO_3)_2$-- were dissolved in distilled water and diluted to 500 cc. 132 grams of dibasic ammonium phosphate --$(NH_4)_2HPO_4$-- were dissolved in distilled water and diluted to 500 cc with heat. The three salt solutions were then combined with heat and stirred for about 10 minutes. The combined solution was vacuum filtered and the resulting precipitate was washed with distilled water and air dried overnight in a static oven at approximately 110°C. The filter cake was broken up into small (1/8 to 1/4 inch) irregular granules for evaluation. The resulting product had a surface pH of 4-5 as determined by acid base indicators and the ratio of strontium to barium in the product was found to be 1 to 3.5.

Example 3

The procedure for preparing the Example 2 was carried out except that 130 grams of $Ba(NO_3)_2$ and 106 grams of $Sr(NO_3)_2$ were dissolved in distilled water in place of the 195 and 53 grams respectively. The product had a surface pH of 4-5 and a Sr/Ba ratio of 2/1 mol/mol. The resulting catalyst was in the form of a fine powder and was deposited on an inert, low surface area Alundum silica-alumina core using a powder-coating step. The step comprised placing the amount of catalyst to be coated into a jar with the Alundum spheres and rotating on a jar-mill for several days to cause the catalyst powder to adhere to the spheres. The resulting coated spheres contained 25% of the active catalyst and 75% inert.

Example 4

212 grams of $Sr(NO_3)_2$ were dissolved in distilled water and diluted to 500 cc. 115 grams of ammonium dihydrogen phosphate --$NH_4H_2PO_4$-- were dissolved in distilled water and diluted to 500 cc. The remaining steps of the catalyst procedure of Example 2 were carried out. The resulting catalyst was believed to contain less than 5% strontium dihydrogen phosphate --$Sr(H_2PO_4)_2$-- with the balance being $SrHPO_4$. The surface pH of this catalyst mixture was 4-4.6 in comparison to substantially pure strontium monohydrogen phosphate which has a surface pH of 4.8-5.4. Substantially pure strontium dihydrogen phosphate was found to have a surface pH of 0.2-1.2; see Example 21.

The product of this example was deposited on the silica-alumina spheres in the same manner as set forth under Example 3.

Example 5

The same procedure for preparing the catalyst of Example 1 was carried out except that 236 grams of calcium nitrate --$Ca(NO_3)_2 \cdot 4H_2O$-- and 115 grams of $NH_4H_2PO_4$ were combined. The resulting dried catalyst particles were coated on the silica-alumina spheres in the same manner as that of Example 3. The analysis of the catalyst formed by this procedure indicated that it consisted essentially of calcium monodihydrogen phosphate with a Ca/P ratio of 1.009 and a surface pH of 4-6. In contrast, substantially pure calcium monohydrogen phosphate had a surface pH of 5-5.5; see Example 16 below. The presence of a very small amount of calcium dihydrogen phosphate may account for the difference in the surface pH value of this catalyst.

Example 6

The catalyst preparation procedure of Example 2 was repeated except that 212 grams of $Sr(NO_3)_2$ were dissolved in place of the mixed barium and strontium nitrate salts and the resulting strontium monohydrogen phosphate catalyst had a surface pH of 4.8-5.2.

Examples 7-15

The following salts were combined and catalysts were prepared in the manner set forth under Example 2:

| Example | Salt Solutions | | Catalyst Formulation | Surface pH |
|---------|----------------|----|----------------------|------------|
| | (a) | (b) | | |
| 7 | 168g. $Nd(NO_3)_3 \cdot 5H_2O$ | 80g. $(NH_4)_2HPO_4$ | $Nd_2(HPO_4)_3$ | * |
| 8 | 217g. $Ce(NO_3)_3 \cdot 6H_2O$ | 99g. $(NH_4)_2HPO_4$ | $Ce_2(HPO_4)_3$ | 0.2-1.2 |
| 9 | 415g. $La(NO_3)_3 \cdot 5H_2O$ | 198g. $(NH_4)_2HPO_4$ | $La_2(HPO_4)_3$ | 0.2-1.8 |
| 10 | 202g. $Sr(NO_3)_2$ + 20 grams of $La(NO_3)_3 \cdot 5H_2O$ | 132g. $(NH_4)_2HPO_4$ | $SrHPO_4/LaHPO_4$ (Sr/La=14.9/1) | 4-5 |
| 11 | 291g. $Co(NO_3)_2 \cdot 6H_2O$ | 132g. $(NH_4)_2HPO_4$ | $CoHPO_4$ | 4.6-4.8 |
| 12 | 291g. $Ni(NO_3)_2 \cdot 6H_2O$ | 132g. $(NH_4)_2HPO_4$ | $NiHPO_4$ | 6.2-6.8 |
| 13 | 242g. $CuCl_2 \cdot 2.5H_2O$ | 132g. $(NH_4)_2HPO_4$ | $CuHPO_4$ | * |
| 14 | 297g. $Zn(NO_3)_2 \cdot 6H_2O$ | 132g. $(NH_4)_2HPO_4$ | $ZnHPO_4$ | 6.2-6.5 |
| 15 | 125g. $Al(NO_3)_3 \cdot 9H_2O$ | 66g. $(NH_4)_2HPO_4$ | $Al_2(HPO_4)_3$ | 2 |

*Colored product, pH N.A.

## Example 16

160 grams of $Ca(NO_3)_4$ were dissolved in distilled water and diluted to 800 cc. 20 cc. of phosphoric acid (88% by wt. in water) were added with agitation. 34.5 cc. of sodium hydroxide solution (50% by wt. NaOH in water) were added to precipitate the $CaHPO_4$ which was filtered, washed, dried and granulated as in Example 2. The resulting product had a surface pH of 5-5.5.

## Controls 1-3

The following salts were also combined in the manner of the Example 1 preparation.

| Control | Salt Solutions | | Catalyst Formulation |
|---------|----------------|---|---------------------|
| | (a) | (b) | |
| 1 | 261g. $Ba(NO_3)_2$ | 230g. $NH_4HSO_4$ | $BaSO_4$ |
| 2 | 75g. CsCl | 40g. $(NH_4)_2HPO_4$ | $CsHPO_4$* |
| 3 | 106g. $Sr(NO_3)_2$ | 40g. 50% NaOH+ 80g. $(NH_4)_2H_2AsO_4$ | $SrHAsO_4$ |

*Did not form a precipitate.

## Control 4

200 grams of $Sr(NO_3)_2$ were dissolved in distilled water and diluted to 400 cc. 92 grams of $H_2SO_4$ were diluted in 200 cc. of distilled $H_2O$. 75 grams of 50 wt. % NaOH solution were diluted to 200 cc. with distilled water. The $H_2SO_4$ and NaOH solutions were mixed together slowly. The $Sr(NO_3)_2$ solution was stirred into the solution containing $H_2SO_4$ and NaOH. The solution was stirred for 10 minutes and the precipitate was filtered, washed and dried. The surface pH of the resulting catalyst was less than 3 which was believed to be substantially all $SrSO_4$.

## Example 17

71 grams of $Na_2HPO_4$ were dissolved in 500 cc. of distilled water. 101.7 grams of $MgCl_2 \cdot 6H_2O$ were dissolved in 500 cc. of distilled $H_2O$. Both solutions were mixed together and the precipitate was filtered, washed and dried. The surface pH of the $MgHPO_4$ product was 7-8.

## Example 18

71 grams of $Na_2HPO_4$ and 130.7 grams of $Ba(NO_3)_2$ were each separately dissolved in 500 cc. of distilled $H_2O$. The 2 solutions were mixed and the precipitate was filtered, washed and dried. The resulting $BaHPO_4$ had a surface pH of 8-9.

Each of the products resulting from the procedures of Examples 17 and 18 above were coated on the silica-alumina spheres in the same manner as indicated in Example 3.

Example 19

The $SrHPO_4$ catalyst of Example 6 was heat treated for 2 hours in the presence of a mixture 20% by volume steam and the balance air at 350°C. The resulting strontium pyrophosphate ($Sr_2P_2O_7$) had a crushing strength of 0.47 kg./mm of length and a packed bulk density of 1.01 kg./l.

Example 20

The $ZnHPO_4$ catalyst product of Example 14 above was coated on the silica-alumina spheres in the manner set forth in Example 3.

Example 21

132.5 grams of strontium hydroxide octahydrate --$Sr(OH)_2 \cdot 8H_2O$-- were dissolved in a solution of 750 cc. of 85% phosphoric acid and 1500 cc. of distilled water. The resulting solution was slowly evaporated to a total volume of about 900 cc. with the temperature being maintained at 25° to 30°C. The solution was cooled to 5°C overnight and a white precipitate was recovered by vacuum filtration. The resulting $Sr(H_2PO_4)_2$ precipitate was washed with 5-300 cc. portions of anhydrous ethanol and with 2-200 cc. portions of anhydrous ether. The product was dried at room temperature under vacuum for 6 hours. An elemental analysis of the product showed a P/Sr mol ratio of 2.04 and the surface pH was found to be 0.2-1.2. The fine powder was pressed into tablets the size of a typical aspirin tablet and crushed to granules 1/8 to 1/4 inch in size.

## Example 22

The fine powder of the catalyst prepared in accordance with Example 21 was deposited on silica-alumina spheres in the manner set forth in Example 3.

## Example 23

2000 grams of $Sr(NO_3)_2$ were dissolved in 2000 cc of deionized water and the solution diluted to 4000 cc with deionized water after dissolution of the $Sr(NO_3)_2$ was complete.

In another container, 1342.3 grams of $Na_2HPO_4$ were dissolved in 2000 cc of deionized water. After solution of the $na_2HPO_4$ was complete, the solution was diluted to 4000 cc with deionized water. The pH of this solution was approximately 8.8.

Precipitation of $SrHPO_4$ was effected by slowly adding the $Na_2HPO_4$ solution to the $Sr(NO_3)_2$ solution with rapid stirring. The white $SrHPO_4$ precipitated rapidly from solution forming a rather thick slurry. This slurry was mixed for one hour, after which time the pH was measured to be about six.

The solid $SrHPO_4$ was recovered by filtering on an eight frame filter press using cloth filters. It was washed with deionized water. After filtering and washing, the solid was dried in a circulating hot air oven at 250°F for four hours. The yield of $SrHPO_4$ was 1680 grams. The solid was wetted and formed into pellets by extrusion through a 3.1 mm die plate and cutting the extrudates to about 1/4 inch in length. After drying the extrudate at 250°F for four hours in a circulating hot air oven, they were heat treated at 662°F for two hours in a 20% steam, 80% air atmosphere.

## Example 24

106 grams of $Sr(NO_3)_2$ and 145 grams of $Ni(NO_3)_2 \cdot 6H_2O$ were dissolved in distilled water and diluted to 500 cc. 132 grams of $(NH_4)_2HPO_4$ were dissolved in distilled water and diluted to 500 cc. the remaining steps of Example 1 were carried out to yield a $(Sr-Ni)HPO_4$ catalyst having a surface pH of 5.4-7.0.

## USE OF CATALYSTS

### Examples 25-62

Each of the products prepared in accordance with Examples 1 through 22 and Controls 1 and 3-5 above were evaluated for catalytic performance for the preparation of TEDA with either a feed mixture containing hydroxyethylpiperazine (HEP) or N-aminoethylpiperazine (AEP) in accordance with the following test procedure:

(a) 20 cc (approximately 6.2g.) of the catalyst was loaded into a 3/4 " diameter stainless steel reactor.

(b) The reactor was placed in a conventional tube furnace such that the catalyst bed was near the furnace center and therefore could be heated to a constant and uniform temperature.

(c) The catalyst bed temperature was raised to a temperature of 340°-400°C over a period of 15 to 30 minutes while a small flow of gaseous nitrogen was maintained through the reactor to aid in the removal of water vapor.

(d) A feed mixture containing HEP and water such that the organic component made up 60% of the mixture was then allowed to flow through the catalyst bed at a rate of 6.5-7.0 cc/hour; the nitrogen flow was discontinued.

(e) The catalyst bed temperature indicated in the tables set forth below was maintained during the run and the product samples were collected and analyzed. Analyses were performed using well-established gas chromatographic techniques.

The yields of TEDA and piperazine (PIP) as well as the conversion obtained from the catalysts of Examples 1-22 in Table 1 can be compared with those of Control Catalysts 1 and 3-5 in Table 2 below.

## TABLE 1
### TEDA PRODUCTION

| Ex-ample | Cat. of Ex. | Form | Nominal Formu-lation | TEDA Yield, Wt. % | PIP Yield, Wt. % | Conver-sion, Mol. % | Temp., °C | Feed |
|---|---|---|---|---|---|---|---|---|
| 25 | 1[1] | Granules | $SrHPO_4$ | 71.5 | 3.7 | 99.3 | 360 | HEP |
| 26 | 1[1] | Granules | $SrHPO_4$ | 80.7 | 3.4 | 99.3 | 360 | HEP |
| 27 | 1 | Granules | $SrHPO_4$ | 71.1 | 7.6 | 95.4 | 370 | HEP |
| 28 | 2 | Granules | $Sr/BaHPO_4$ (Sr/Ba=1/3.5) | 83.8[2] | 7.0[3] | 99.9 | 360 | HEP |
| 29 | 3 | 25% Coated Spheres | $Sr/BaHPO_4$ (Sr/Ba=2/1) | 67.8 | 5.5 | 99.1 | 380 | HEP |
| 30 | 3 | 25% Coated Spheres | $Sr/BaHPO_4$ (Sr/Ba=2/1) | 13.8 | 9.1 | 56.8 | 400 | AEP |
| 31 | 4 | 25% Coated Spheres | $SrHPO_4/$ $Sr(H_2PO_4)_2$ | 63.5 | 14.7 | 98.3 | 380 | HEP |
| 32 | 4 | 25% Coated Spheres | $SrHPO_4/$ $Sr(H_2PO_4)_2$ | 18.8 | 16.4 | 77.0 | 400 | AEP |
| 33 | 5 | 25% Coated Spheres | $CaHPO_4/$ $Ca(H_2PO_4)_2$ | 56.2 | 5.8 | 91.2 | 380 | HEP |

[1] Based on average of two duplicate runs.

[2] 97.3 mol.% based on feed.

[3] 10.6 mol.% base on feed.

TABLE 1 (cont'd)

TEDA PRODUCTION

| Ex-ample | Cat. of Ex. | Form | Nominal Formu-lation | TEDA Yield, Wt. % | PIP Yield, Wt. % | Conver-sion, Mol. % | Temp., °C | Feed |
|---|---|---|---|---|---|---|---|---|
| 34 | 5 | 25% Coated Spheres | $CaHPO_4/$ $Ca(H_2PO_4)_2$ $^{(4)}$ | 34.4 | 9.9 | 90.2 | 380 | AEP |
| 35 | 6 | Granules | $SrHPO_4$ $^{(4)}$ | 76.0 | 7.8 | 98.6 | 360 | HEP |
| 36 | 6 | Granules | $SrHPO_4$ | 29.0 | 21.0 | 99.0 | 400 | AEP |
| 37 | 7 | Granules | $Nd_2(HPO_4)_3$ | 18.2 | 5.4 | 98.4 | 340 | HEP |
| 38 | 7 | Granules | $Nd_2(HPO_4)_3$ | 7.5 | 2.1 | 92.6 | 360 | AEP |
| 39 | 8 | Granules | $Ce_2(HPO_4)_3$ | 32.3 | 8.4 | 99.9 | 340 | HEP |
| 40 | 8 | Granules | $Ce_2(HPO_4)_3$ | 7.8 | 7.9 | 92.6 | 360 | AEP |
| 41 | 9 | Granules | $La_2(HPO_4)_3$ | 16.2 | 4.2 | 98.8 | 340 | HEP |
| 42 | 9 | Granules | $La_2(HPO_4)_3$ | 25.5 | 3.3 | 95.2 | 380 | AEP |
| 43 | 10 | Granules | $La/SrHPO_4$ (La/Sr=1/15) | 58.7 | 5.3 | 99.2 | 360 | HEP |
| 44 | 10 | Granules | $La/SrHPO_4$ (La/Sr=1/15) | 27.4 | 14.6 | 96.1 | 380 | AEP |
| 45 | 11 | Granules | $CoHPO_4$ | 40.0 | 8.4 | 98.7 | 360 | HEP |
| 46 | 11 | Granules | $CoHPO_4$ | 19.4 | 17.4 | 91.9 | 380 | AEP |
| 47 | 12 | Granules | $NiHPO_4$ | 36.2 | 5.6 | 82.4 | 360 | HEP |

$^{(4)}$Analysis of catalyst after reaction to produce TEDA indicates the formation of a minor amount of $SrP_2O_7$.

TABLE 1 (cont'd)

TEDA PRODUCTION

| Example | Cat. of Ex. | Form | Nominal Formulation | TEDA Yield, Wt. % | PIP Yield, Wt. % | Conversion, Mol. % | Temp., °C | Feed |
|---|---|---|---|---|---|---|---|---|
| 48 | 12 | Granules | $NiHPO_4$ | 6.0 | 7.8 | 56.7 | 380 | AEP |
| 49 | 13 | Granules | $CuHPO_4$ (5) | 17.6 | 4.2 | 99.4 | 340 | HEP |
| 50 | 14 | Granules | $ZnHPO_4$ | 45.9 | 6.7 | 94.7 | 380 | HEP |
| 51 | 15 | Granules | $Al_2(HPO_4)_3$ | 39.7 | 9.4 | 98.3 | 380 | HEP |
| 52 | 15 | Granules | $Al_2(HPO_4)_3$ | 17.1 | 25.8 | 88.4 | 380 | AEP |
| 53 | 16 | Granules | $CaHPO_4$ | 59.2 | 23.5 | 99.6 | 360 | HEP |
| 54 | 17 | 25% Coated Spheres | $MgHPO_4$ | 47.2 | 15.8 | 99.9 | 380 | HEP |
| 55 | 18 | 25% Coated Spheres | $BaHPO_4$ | 23.2 | 1.0 | 37.4 | 380 | HEP |
| 56 | 19 | Granules | $Sr_2P_2O_7$ | 30.7 | 9.0 | 98.4 | 340 | HEP |
| 57 | 19 | Granules | $Sr_2P_2O_7$ | 41.0 | 9.0 | 99.2 | 360 | HEP |
| 58 | 20 | 25% Coated Spheres | $ZnHPO_4$ | 12.2 | 11.3 | 57.9 | 400 | AEP |
| 59 | 21 | Granules | $Sr(H_2PO_4)_2$ | 24.7 | 4.4 | 95.5 | 320 | HEP |
| 60 | 21 | Granules | $Sr(H_2PO_4)_2$ | 6.7 | 0.6 | 99.6 | 320 | AEP |
| 61 | 22 | 25% Coated Spheres | $Sr(H_2PO_4)_2$ | 22.0 | 3.6 | 98.9 | 320 | HEP |
| 62 | 22 | 25% Coated Spheres | $Sr(H_2PO_4)_2$ | 10.9 | 18.3 | 91.1 | 320 | AEP |

(5) Analysis of catalyst after reaction to produce TEDA indicated the formation of minor amounts of $CuP_2O_7$ and elemental Cu.

TABLE 2

| Catalyst of Control | Form | Nominal Formu-lation | TEDA Yield, Wt. % | PIP Yield, Wt. % | Conversion, Mol. % | Temp., °C | Feed |
|---|---|---|---|---|---|---|---|
| 1 | Granules | $BaSO_4$ | 14.3 | 2.9 | 31.2 | 360 | HEP |
| 3 | 25% Coated Spheres | $SrHA_sO_4$ | 4.6 | 6.2 | 36.2 | 340 | HEP |
| 3 | 25% Coated Spheres | $SrHA_sO_4$ | 1.2 | 1.6 | 43.5 | 360 | AEP |
| 4 | Granules | $SrSO_4$ | 2.1 | 27.3 | 36.4 | 360 | HEP |
| 5[6] | Granules | $AlPO_4$ | 28.4 | 6.0 | 100 wt.% | 400 | HEP |
| 5[7] | Granules | $AlPO_4$ | 33.9 | 26.7 | 83 wt.% | 375 | AEP |

[6] Data obtained from Example II of U.S. Patent 3,297,701.

[7] Data obtained from Example XXI of U.S. Patent 3,297,701.

The results set forth in Table 1 where the catalysts of the examples demonstrate a unique ability to convert over 50 mol.% of the feed to products the majority of which is TEDA. Depending on the particular feed stock, the yield of TEDA ranged from 6% in the worst case using Ni to approximately 84% in the best mode using 1 part $SrHPO_4$ to 3.5 parts $BaHPO_4$, see Example 1 above. The former being the use of $NiHPO_4$ with an aminoethyl-piperazine feed. When the feed over $NiHPO_4$ was changed to hydroxyethylpiperazine, the yield increased 6 fold with a corresponding increase in the conversion from approximately 57% to 82%. The $BaHPO_4$ catalysts per se did not meet the criteria for an effective catalyst for the conversion of HEP to TEDA. However, when approximately four parts of this catalyst were combined with one part of $SrHPO_4$ as in the Example 2, the yield of TEDA and the conversion increased based on a synergistic effect over that of the $SrHPO_4$ catalyst.

Example 63

The catalyst of Example 23 was tested in the conversion of crude HEP to TEDA. The reaction was carried out at atmospheric pressure, at a liquid hourly space velocity of 0.3 and at the temperatures indicated in Table 3 below.

TABLE 3

|  | Initial | After 78 Days |
|---|---|---|
| Bed Temp., °C | 360 | 368 |
| HEP Conversion, wt. % | 99+ | 99+ |
| TEDA Yield, wt. % | 40.5 | 43.0 |
| PIP Yield, wt. % | 13.5 | 18.5 |

Example 64

The catalyst of Example 23 was tested for the conversion of diethanolamine to TEDA. The test was carried out at 370°C using a feed consisting of diethanolamine and water (2.0 to 1.0 mole ratio) pumped into the reactor at a rate of 4.4 liquid cc/hr along with helium diluent at a rate of 25 cc/minute. The diethanolamine was in completely converted to TEDA, as the only, i.e. about 26 mol. %, recovered product.

Example 65

A 64% by weight solution of N-aminoethyl piperazine in water was passed over a catalyst composition consisting essentially of $SrHPO_4$ at 380°C and at a liquid hourly space velocity of 0.3 volumes of liquid per volume of catalyst. In a first pass operation there was obtained 96.8 mol. % conversion of the feed compound, obtaining a yield of 34.8% by weight (40.1 mol. %) TEDA and 27.1% by weight (40.6 mol. %) PIP.

Other typical condensation reactions in which $SrHPO_4$ may be employed as a catalyst include the formation of amines by amination of the corresponding alcohols with ammonia and the formation of polyamines from glycols and diamines.

It is believed that the key to the properties of the $SrHPO_4$ as a highly selective catalyst is due to the presence of a specific structure, which provides a narrow range of acidity. This narrow acidity range displayed by $SrHPO_4$ may be optimum for promoting certain types of acid catalyzed reactions, in contrast to such catalysts as alumina, silica-alumina and the like which have acid sites of widely varying strength, and hence show relatively low selectivity for the desired reaction.

Example 66

Diethyleneglycol was passed over the $SrHPO_4$ catalyst of Example 23 in the presence of water at a temperature of 370°C and at a contact time of 6.7 seconds. The feed contained 57 vol. % diethyleneglycol and 43 vol. % $H_2O$. The reaction product contained 33 wt. % 1,4-dioxane, corresponding to a yield of 47 mol. %.

The addition of water to the organic feeds may be desirable to prevent loss of catalyst activity as a result of dehydration of the $SrHPO_4$ to the pyrophosphate.

## Example 67

The $SrHPO_4$ catalyst of Example 23 was tested for the conversion of 1, 4-butanediol to tetrahydrofuran. The test was carried out at 350°C using a feed consisting of 20 percent by volume of water and 80 percent by volume of 1, 4-butanediol pumped to the tubular reactor at a rate of 4.4 cc/hr. Helium dilutent was also fed at the rate of 30 cc/min. Under these conditions, the diol was completely converted to tetrahydrofuran.

## Example 68

The $CaHPO_4$ catalyst of Example 5 was recovered in a fine, powdered state and was deposited on inert alumina spheres instead of the silica-alumina spheres in the same manner as set forth under Example 3. 20 cc of the resulting coated alumina contained 2 gms. of $CaHPO_4$. The performance of this catalyst was evaluated for the preparation of an alipathic secondary amine with a feed mixture of monoethylamine (EA) and methanol using the general procedure used in Examples 25-62 for the preparation of TEDA. Specifically, 1 mol. of the primary amine and 1 mol. of the alcohol were reacted at 350°C, 1 atmosphere pressure and an LHSV of 0.15/hr. The conversion was 23.9 mol. % of the ME to the secondary aliphatic amine. The yield of methylethylamine (MEA) was 16.5 mol. % of the amine feed with a selectivity of 69 mol. %. The only other product in any significant quantity was dimethylethylamine (DMEA) with a yield of 5.4 mol. % and a selectivity of 22 mol %.

Example 69

The procedure of Example 68 was followed except that 1 mol. of diethylamine (DEA) was substituted for 1 mol. of EA. 27.6 mol. % of this primary amine in the feed was converted for the most part to a single secondary amine, i.e. diethylmethylamine (DEMA) and a trace amount of a tertiary amine, i.e. triethylamine. The yield of DEA fed was 23 mol. % DEMA with a selectivity of 83.3 mol. %.

Examples 70-71

3 grams of the $La_2(HPO_4)_3$ catalyst of Example 9 were coated onto alumina spheres and the procedures of Example 60 and 69 were followed. The results from these reactions are summarized in Table 4 below.

Examples 72-73

The $SrHPO_4$ catalyst of Example 6, was used to convert methanol and either monoethylamine (Example 72) or diethylamine (Example 73) in the same manner set forth under Example 68.

The results of these Examples 72-73 are summarized and compared with the other Examples 68-71 in Table 4 below.

TABLE 4

ALIPHATIC AMINE PRODUCTION

| | Amine Feed | Yield, mol. % | | | Selectivity, Mol. % | | | Conversion Mol. % |
|---|---|---|---|---|---|---|---|---|
| | | MEA | DMEA[7] | DEMA | MEA | DMEA | DEMA | |
| Example | | | | | | | | |
| 68 | EA | 16.5 | 5.4 | - | 69 | 22 | - | 23.9 |
| 69 | DEA | - | - | 23 | - | - | 83.3 | 27.6 |
| 70 | EA | 27 | 25 | - | 47 | 44 | - | 57 |
| 71 | DEA | - | - | 39 | - | - | 66 | 59 |
| 72 | EA | 22 | 17 | - | 55 | 17 | - | 40 |
| 73 | DEA | 1.4 | 6.9 | 41 | 2.8 | 13.8 | 82 | 50 |

[7]Dimethylethylamine.

The data of Table 4 above illustrates the unexpectedly high selectivities to the corresponding aliphatic amine without the formation of the corresponding byproducts of thermodynamic amine equilibration. The relatively low conversion to product of Examples 68-71 can be attributed to the fact that only 2 gms. of $CaHPO_4$ or 3 gms. $La_2(HPO_4)_3$ were used in Examples 68-71 in comparison to about 20 gms. of $SrHPO_4$ that were used in Examples 72 and 73.

Example 74

The $CuHPO_4$ catalyst of Example 13 was coated onto alumina spheres and the procedure of Example 68 was followed except that 1 mol. of ammonia was substituted for 1 mol. of EA. The results from this reaction were a 65 mol. % yield of monomethylamine (MA) based on the methanol, an 87 mol.% selectivity to MA and a methanol conversion of 75 mol. %.

Example 75

The $La_2(HPO_4)_3$ catalyst of Example 9 was deposited on alumina spheres and the procedure of Example 74 was followed. The results were yields based on the methanol in the feed of 13 mol. % trimethylamine (TMA), 2.8 mol. % dimethylamine (DMA) and 3 mol. % monomethylamine (MA), selectivities based on the methanol of 69 mol. % TMA, 14.9 mol. % DMA and 16 mol. % MA, and a methanol conversion of 52 mol. %.

It has also been found that $MgHPO_4$ and $BaHPO_4$ were effective in selectivity converting amines to the corresponding higher molecular weight amine.

Examples 76-82

The $(Sr-Ni)HPO_4$ catalyst of Example 24 was evaluated for the preparation of N-(2-dimethylaminoethyl)morpholine (DMAEM) with a feed mixture of morpholine (MOR), dimethyl-

0111928

ethanolamine (DMEA), distilled water, hydrogen and helium in the amounts shown below in Table 5 using the general procedure used in Examples 25-62 for the TEDA preparation. Specifically the condensation reaction was carried out at 340°C, 1 atmosphere of pressure and an LHSV in the range of 0.31-0.44/hr. as shown in Table 5 in the presence of 20 cc. of the $(Sr-Ni)HPO_4$ catalyst granules.

Examples 83-86

The $SrHPO_4$ catalyst of Example 6 was used in place of the $(Sr-Ni)HPO_4$ catalyst of Example 24 and the condensation reactions were carried out in the same manner as described under Examples 76-82. The results of these runs are summarized and compared with the Examples 76-82 feed, yield and conversion data in Table 5 below.

TABLE 5

DMAEM PRODUCTION

| Example | Feed, Vol. % | LHSV, hr.$^{-1}$ | Yield, Mol. % MOR | Conversion, Mol. % |
|---------|--------------|------------------|-------------------|--------------------|
| 76 | MOR 60<br>DMEA 20<br>$H_2O$ 20<br>He (20 cc/min)<br>$H_2$ (20 cc/min) | 0.31 | 100 | 16 |
| 77 | MOR 60<br>DMEA 20<br>$H_2O$ 20<br>He (20 cc/min)<br>$H_2$ (20 cc/min) | 0.44 | 63 | 23 |
| 78 | MOR 40<br>DMEA 40<br>$H_2O$ 20<br>He (20 cc/min)<br>$H_2$ (20 cc/min) | 0.31 | 53 | 43 |

0111928

## TABLE 5 - (Cont'd)
### DMAEM PRODUCTION

| Example | Feed, Vol. % | LHSV, hr.$^{-1}$ | Yield, Mol. % MOR | Conversion, Mol. % |
|---|---|---|---|---|
| 79 | MOR 40<br>DMEA 40<br>$H_2O$ 20<br>He (20 cc/min)<br>$H_2$ (20 cc/min) | 0.21 | 58 | 48 |
| 80 | MOR 20<br>DMEA 60<br>$H_2O$ 20<br>He (20 cc/min)<br>$H_2$ (20 cc/min) | 0.31 | 52 | 60 |
| 81 | MOR 20<br>DMEA 60<br>$H_2O$ 20<br>He (20 cc/min)<br>$H_2$ (20 cc/min) | 0.21 | 58 | 75 |
| 82 | MOR 40<br>DMEA 20<br>$H_2O$ 40<br>He (20 cc/min)<br>$H_2$ (20 cc/min) | 0.31 | 97 | 21 |
| 83 | MOR 40<br>DMEA 20<br>$H_2O$ 20<br>He (20 cc/min) | 0.21 | 42 | 32 |
| 84 | MOR 40<br>DMEA 40<br>$H_2O$ 20<br>He (20 cc/min) | 0.21 | 37 | 28 |
| 85 | MOR 60<br>DMEA 20<br>$H_2O$ 20<br>He (20 cc/min) | 0.21 | 43 | 22 |
| 86 | MOR 20<br>DMEA 60<br>$H_2O$ 20<br>He (20 cc/min) | 0.21 | 33 | 45 |

Examples 87-91

The catalyst product of Example 1 was evaluated for catalytic performance for the preparation of morpholine from diglycolamine at 1 atmosphere in accordance with the following test procedure:

(a) 10 cc (approximately 3.1 g.) of $SrHPO_4$ was loaded into a 3/4 " diameter stainless steel reactor.

(b) The reactor was placed in a conventional tube furnace such that the catalyst bed was near the furnace center and therefore could be heated to a constant and uniform temperature.

(c) The catalyst bed temperature was slowly raised to a temperature of 250°C over a period of 15 to 30 minutes while a small flow of gaseous helium was maintained through the reactor in three of the examples.

(d) A feed mixture containing DGA and water (except for Examples 88 and 90) in the ratio set forth in Table 6 below was then allowed to flow through the catalyst bed at an LHSV of 0.21 to 0.88; the helium flow was continued through the run (except for Examples 90-91).

(e) The catalyst bed temperature indicated in Table 6 below were maintained throughout the run and the product samples were collected and analyzed. Analyses were performed using well-established gas chromatographic techniques.

The operating conditions and yields obtained from the catalyst of Example 1 are summarized in Table 6 below.

## Table 6

| Example | 87 | 88 | 89 | 90 | 91 |
|---|---|---|---|---|---|
| Feed, DGA/$H_2O$, Vol. % | 80/20 | 100/0 | 80/20 | 100/0 | 50/50 |
| Helium Diluent, cc/min. | 34.5 | 34 | 28.5 | None | None |
| Temp., °C | 320 | 320 | 370 | 350 | 330 |
| Contact Time, sec. | 12 | 55 | 12 | 13 | 22 |
| LHSV | 0.21 | 0.21 | 0.44 | 0.88 | 0.21 |
| Yield of Morpholine, mol. % | 85 | 63 | 37 | 46 | 75 |
| Selectivity to Morpholine % | 85 | 74 | 37 | 57.5 | 75.6 |
| Conv. of DGA mol. % | 100 | 85 | 100 | 80 | 99 |
| Dioxane Yield, mol. % | ← | — less than 1% | — → | | |

In each of the Examples 87-91, it was unexpectedly found that DGA could be to selectively converted to morpholine without the conversion of appreciable quantities of dioxane, tar or other high molecular weight components. It would be expected that the reaction product from DGA conversion would contain substantially equal amounts of dioxane and morpholine.

### Examples 92-118

The test procedure set forth in Examples 87-91 was followed in Examples 92-118 in the presence of the Example 1 catalyst. Table 7 below sets forth the feed mixture, operating conditions and the product yields for each example.

## TABLE 7

| Example | DGA/$H_2O$, Vol.% | Temp. °C | LHSV | Helium Diluent, cc/min. | Conversion of DGA, mol.% | Morpholine yield, mol.% | Morpholine % Selectivity |
|---|---|---|---|---|---|---|---|
| 92 | 100/0 | 370°C | 0.44 | None | 99 | 36 | 36 |
| 93 | 100/0 | 370°C | 0.88 | None | 99 | 47 | 47 |
| 94 | 100/0 | 370°C | 1.3 | None | 94 | 48 | 51 |
| 95 | 100/0 | 350°C | 0.44 | None | 93 | 47 | 51 |
| 96 | 100/0 | 340°C | 0.21 | None | 95 | 51 | 54 |
| 97 | 100/0 | 330°C | 0.21 | None | 100 | 53 | 53 |
| 98 | 100/0 | 320°C | 0.88 | None | 83 | 43 | 52 |
| 99 | 80/20 | 370°C | 0.44 | None | 100 | 43 | 43 |
| 100 | 80/20 | 350°C | 0.44 | None | 93 | 57 | 61 |
| 101 | 80/20 | 350°C | 0.44 | 28.5 | 100 | 55 | 55 |
| 102 | 80/20 | 340°C | 0.67 | 28.5 | 93 | 49 | 53 |
| 103 | 80/20 | 330°C | 0.44 | 28.5 | 95 | 57 | 60 |
| 104 | 80/20 | 330°C | 0.31 | 28.4 | 100 | 68 | 68 |
| 105 | 80/20 | 325°C | 0.21 | None | 94 | 47 | 50 |
| 106 | 80/20 | 320°C | 0.31 | 28.5 | 95 | 73 | 77 |
| 107 | 80/20 | 320°C | 0.21 | 28.5 | 100 | 81 | 81 |
| 108 | 80/20 | 320°C | 0.44 | 28.5 | 90 | 46 | 51 |
| 109 | 80/20 | 320°C | 0.14 | 28.5 | 100 | 73 | 73 |
| 110 | 80/20 | 320°C | 0.67 | None | 79 | 30 | 38 |
| 111 | 80/20 | 320°C | 0.31 | None | 86 | 35 | 41 |
| 112 | 80/20 | 310°C | 0.44 | 28.5 | 82 | 38 | 46 |
| 113 | 50/50 | 320°C | 0.31 | None | 77 | 46 | 60 |
| 114 | 50/50 | 320°C | 0.21 | None | 97 | 72 | 74 |
| 115 | 65/35 | 320°C | 0.21 | None | 98 | 54 | 55 |
| 116 | 65/35 | 330°C | 0.31 | None | 79 | 50 | 63 |
| 117 | 35/65 | 325°C | 0.31 | None | 73 | 54 | 74 |
| 118 | 35/65 | 325°C | 0.21 | None | 100 | 60 | 60 |

What Is Claimed Is:

1.   In methods for the synthesis of organic compounds by condensation reactions in the presence of phosphate catalysts, the improvement which comprises the use as such catalysts which are selected from the group consisting of the pyrophosphate, monohydrogen phosphate and dihydrogen phosphate of strontium, copper, magnesium, calcium, barium, zinc, lanthanum, aluminum, cobalt, nickel, cerium, neodymium, and mixtures thereof.

2.   The method as defined in Claim 1 wherein said catalyst is associated with a carrier of the group consisting of silica, alumina and silica-alumina.

3.   The method as defined in Claim 1 wherein such condensation reaction is one resulting in the elimination of water.

4.   The method as defined in Claim 1 wherein such condensation reaction is one resulting in the elimination of ammonia.

5.   The method as defined in Claim 1 or 2 wherein such condensation reaction is one effecting cyclization.

6.   The method as defined in Claim 1 wherein such condensation reaction comprises the production of triethylenediamine from hydroxyethylpiperazine.

7.   The method as defined in Claimed 1 wherein such condensation reaction comprises the production of triethylenediamine from crude hydroxyethylpiperazine.

8.   The method as defined in Claim 1 wherein such reaction comprises the production of triethylenediamine from ethanolamines.

9. The method as defined in Claim 1 wherein such condensation reaction comprises the production of triethylenediamine from N-aminoethylpiperazine.

10. The method as defined in Claim 1 wherein such condensation reaction comprises the production of symmetrical and unsymmetrical aliphatic or aromatic amines from an alcohol and an amine or ammonia.

11. The method as defined in Claim 1 wherein such condensation reaction comprises the production of dimethylaminoethylmorpholine.

12. The method as defined in Claim 1 wherein such condensation reaction comprises the production of a morpholine compound selected from the group consisting of morpholine and alkyl morpholine.

13. The process of Claims 11 or 12 wherein the conversion takes place in the presence of an inert gas.

14. The method as defined in any of Claims 6, 7, 9, 11 or 12 wherein the reaction is carried out in the presence of water.

15. A method which comprises converting a substituted piperazine compound selected from the group consisting of hydroxyethylpiperazine and aminoethylpiperazine to triethylenediamine at a temperature in the range of about 285°C to 420°C, a pressure in the range of above 1,5 and up to 150 atmospheres, a liquid hourly space velocity of about 0.05 to 1.5 in the presence of a catalyst selected from the group consisting of the pyrophosphate, monohydrogen phosphate and dihydrogen phosphate of strontium, copper, magnesium, calcium, barium, zinc, lanthanum, aluminum, cobalt, nickel, cerium, neodymium, and mixtures thereof.

16.  A method which comprises converting hydroxyethylpiperazine to greater than 50 mol.% yield of triethylenediamine at a temperature in the range of about 340° to 400°C, a pressure in the range of about 3 to 10 atmospheres, a liquid hourly space velocity of about 0.1 to 0.3 in the presence of a catalyst selected from the group consisting of the monohydrogen phosphate and dihydrogen phosphate of strontium, calcium, magnesium, zinc, mixtures of strontium and barium in the ratio of Sr to Ba of 1 to 5 to 5 to 1 and mixtures of lanthanum and strontium in the ratio of La to Sr of 15 to 1 to 1 to 15.

17.  A method which comprises converting a nitrogen-containing compound selected from the group consisting of ammonia, primary and secondary aliphatic amines and aromatic amines to its corresponding symmetrical and unsymmetrical higher molecular weight aliphatic or aromatic amine in the presence of an alcohol at a temperature in the range of about 285°C to 420°C, a pressure in the range of above 1,5 and up to 150 atmospheres, a liquid hourly space velocity of about 0.05 to 1.5 in the presence of a catalyst selected from the group consisting of the pyrophosphate, monohydrogen phosphate and dihydrogen phosphate of strontium, copper, magnesium, calcium, barium, zinc, lanthanum, aluminum, cobalt, nickel, cerium, neodymium, and mixtures thereof.

18.  A method which comprises selectively converting greater than 50 mol. % of a nitrogen-containing compound selected from the group consisting of ammonia, primary and secondary aliphatic amines and aromatic amines having 1 to 20 cartons per molecule to its corresponding higher molecular weight aliphatic or aromatic amine in the presence of an alcohol having 1 to 20 carbon atoms per molecule in the molar ratio of said nitrogen-containing compound to alcohol in the range of about 6 to 1 to 1 to 6 at a temperature in the range of about 340°C to 400°C, a pressure in the range of above 1,5 and up to 150 atmospheres, a liquid hourly space velocity of 0.1 to 0.3 in the presence of a catalyst selected from the group consisting of the pyrophosphate, monohydrogen phosphate and dihydrogen phosphate of strontium, copper, magnesium, calcium, and barium, lanthanum, and mixtures thereof.

19. A method which comprises converting morpholine and dimethylethanolamine to dimethylaminoethylmorpholine in the presence of water and hydrogen at a temperature in the range of about 285°C to 420°C, a pressure in the range of above 1,5 and up to 150 atmospheres, a liquid hourly space velocity of about 0.05 to 1.5 in the presence of a catalyst selected from the group consisting of the pyrophosphate, monohydrogen phosphate and dihydrogen phosphate of strontium, copper, magnesium, calcium, barium, zinc, lanthanum, aluminum, cobalt, nickel, cerium, neodymium, and mixtures thereof.

20. The method as defined in Claim 19 wherein an inert gas is present during the conversion.

21. A method which comprises converting morpholine and dimethylethanolamine in the molar ratio of morpholine to dimethylethanolamine of about 1 to 3 to 3 to 1 in the presence of water, hydrogen and an inert gas to greater than 50 mol. % yield of dimethylaminoethylmorpholine at a temperature in the range of about 360°C to 400°C, a pressure in the range of about 3 to 10 atmospheres, a liquid hourly space velocity of about 0.1 to 0.3 in the presence of a mixed nickel and strontium hydrogen phosphate catalyst.

22. A process which comprises converting a diglycolamine compound to a morpholine compound at a temperature in the range of about 285°C to 420°C, a pressure in the range of above 1,5 and up to 150 atmospheres, a liquid hourly space velocity of about 0.05 to 1.5 in the presence of a catalyst selected from the group consisting of the pyrophosphate and the monohydrogen and dihydrogen phosphate of strontium, copper, magnesium, calcium, barium, zinc, lanthanum, aluminum, cobalt, nickel, cerium, neodymium, and mixtures thereof.

23. The process of Claims 22 wherein the conversion takes place in the presence of an inert gas.

24. The process of Claim 22 wherein said diglycolamine compound is selected from the group consisting of diglycolamine and alkyl diglycolamine and mixtures thereof and said morpholine compound is selected from the group consisting of morpholine and alkyl morpholine and mixtures thereof, wherein each of said alkyl groups contains from 1 to 6 carbon atoms.

25. A process which comprises converting diglycolamine to morpholine at a temperature in the range of about 285°C to 420°C, a pressure in the range of above 1,5 and up to 150 atmospheres, a liquid hourly space velocity of about 0.05 to 1.5 in the presence of a catalyst selected from the group consisting of the pyrophosphate and the monohydrogen and dihydrogen phosphate of strontium, copper, magnesium, calcium, barium, zinc, lanthanum, aluminum, cobalt, nickel, cerium, neodymium, and mixtures thereof.

26. A process which comprises converting diglycolamine to greater than 50 mol.% yield of morpholine and less than 1 mol.% yield of dioxane at a temperature in the range of about 300° to 370°C, a pressure in the range of about 3 to 10 atmospheres a liquid hourly space velocity of about 0.1 to 1.0 in the presence of a catalyst consisting essentially of strontium monohydrogen phosphate.

27. The method as defined in Claims 25 or 26, wherein the conversion is carried out in the presence of water.

28. The method as defined in any of Claims 25, 26 or 27 wherein the conversion takes place in the presence of an inert gas.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| P,X | EP-A-0 069 322 (AIR PRODUCTS) * Claims * ----- | 1-28 | C 07 C 85/06<br>C 07 D 295/02<br>C 07 D 295/12<br>C 07 D 307/08<br>C 07 D 319/12<br>C 07 D 487/08<br>B 01 J 27/18 //<br>(C 07 D 487/08<br>C 07 D 241/00<br>C 07 D 241/00 ) |

TECHNICAL FIELDS
SEARCHED (Int. Cl. ³)

C 07 C 85/00
C 07 D 295/00
C 07 D 307/00
C 07 D 319/00
C 07 D 487/00

The present search report has been drawn up for all claims

| Place of search | Date of completion of the search | Examiner |
|---|---|---|
| THE HAGUE | 16-03-1984 | MOREAU J.M. |